# EUROPÄISCHE PATENTSCHRIFT

(11) **EP 2 957 251 B1**
(45) Veröffentlichungstag und Bekanntmachung des Hinweises auf die Patenterteilung: **05.08.2020**
(21) Anmeldenummer: 15172938.1
(22) Anmeldetag: 19.06.2015
(51) Int. Cl.: A61C 1/08, A61C 9/00, A61C 13/00

(54) **VORRICHTUNG ZUR VERWENDUNG IN EINEM VERFAHREN ZUM HERSTELLEN EINER ZAHNIMPLANTATSTRUKTUR**
DEVICE FOR USE IN A METHOD FOR THE PRODUCTION OF A DENTAL IMPLANT STRUCTURE
DISPOSITIF D'UTILISATION DANS UN PROCÉDÉ DE FABRICATION D'UNE STRUCTURE D'IMPLANT DENTAIRE

(30) Priorität: 19.06.2014 DE 202014004912 U; 09.03.2015 DE 202015001753 U
(43) Veröffentlichungstag der Anmeldung: 23.12.2015
(60) Teilanmeldung: 20173298.9
(73) Patentinhaber: R+K CAD CAM Technologie GmbH & Co. KG, 12681 Berlin (DE)
(72) Erfinder: Holzhausen, Stefan, 01705 Freital (DE); Sembdner, Philipp, 01069 Dresden (DE); Ellmann, Daniel, 15370 Fredersdorf (DE); Klar, Andreas, 12683 Berlin (DE)
(74) Vertreter: Eisenführ Speiser

(56) Entgegenhaltungen:
- EP-A1- 2 072 018
- WO-A1-2013/106430
- US-A1- 2013 316 298

## Beschreibung

Die Erfindung betrifft eine Vorrichtung zur Verwendung in einem Verfahren zum Herstellen einer Zahnimplantatstruktur sowie ein Verfahren zur Herstellung einer Zahnimplantatstruktur.

Bei der durchgängig rechnergestützten Planung einer dentalen Versorgung, die anhand von individuellen 3D-Patientenvolumendaten erfolgt, besteht die Problemstellung in der kontinuierlichen Informationsversorgung und -transformation von der Planung am Patientendatensatz bis hin zur Fertigung. Bisher praktizierte Abläufe leiden an einem informationellen Bruch in der digitalen Prozesskette. Die Planungsdaten (z.B. Position und Orientierung von Implantaten und Bohrhülsen) liegen zunächst im CT-Koordinatensystem vor und können somit nicht direkt für die Fertigung verwendet werden. Sie müssen in ein einheitliches und bekanntes Koordinatensystem überführt werden, um einen zweckmäßigen durchgängig digitalen Workflow zu erzielen. Notwendige Referenzobjekte für diese Transformation müssen dabei sowohl im 3D-Volumendatensatz, als auch im Datensatz der Positionierschablone bekannt sein.

Die US 2013/0316298 A1, die den nächstkommenden Stand der Technik bildet, offenbart eine Vorrichtung zur Verwendung in einem Verfahren zum Herstellen einer Zahnimplantatstruktur, mit einer Abtasteinrichtung, die den Kiefer eines Patienten abtastet und daraus entsprechende Bilddaten erzeugt, mit einer ersten Verarbeitungseinrichtung, die in den Bilddaten aufgrund einer Festlegung einer Position, einer Orientierung und eines Raumes für die Anordnung eines Implantates Implantatdaten erzeugt, mit einer zweiten Verarbeitungseinrichtung, die die Implantatdaten mit abgespeicherten Daten einer Vielzahl von unterschiedlichen Implantatausführungen vergleicht und als Ergebnis dieses Vergleiches mindestens eine passende Implantatausführung aus der Vielzahl der unterschiedlichen Implantatausführungen angibt, und mit einer Transformationseinrichtung, die die Implantatdaten auf der Grundlage eines Referenzkoordinatensystems in Maschinendaten für eine Bearbeitungsmaschine transformiert, die für das Einsetzen des Implantates in den Kiefer verwendet wird.

Patientenindividuelle Bohrhilfen zur passgenauen Implantation von Zahnimplantaten gelten als Stand der Technik (EP 1 101 451 A2). Zur Herstellung individueller Positionierschablonen für die Dentalimplantation erfolgt die manuelle Anfertigung einer Scanschablone mit sog. Scanzähnen über einen analogen oder digitalen Zahnabdruck. In diese Schablone wird vom Zahntechniker ein Referenzkörper (Referenzplatte, Referenzpins etc.) eingearbeitet. Nach der Digitalisierung des Patientenunterkiefers und/oder -oberkiefers mit aufgesetzter Scanschablone mittels Oberflächenscan und/oder bildgebender Verfahren (CT, DVT) erfolgt eine rechnergestützte Implantatplanung (EP 2 072 018 A1). Über den Referenzkörper in Form einer Platte erfolgt eine interaktive Ausrichtung der Implantate zum Koordinatensystem der Positionierschablone. Zum gegenwärtigen Zeitpunkt gibt es im weiteren Verlauf der Prozesskette zwei prinzipielle Vorgehensweisen. Zum einen kann der Datensatz direkt online an den entsprechenden Systemanbieter versendet werden. Nach 5 bis 10 Tagen erhält der Zahntechniker die zentral gefertigte Positionierschablone. Es wird somit zusätzlich zur Scanschablone die eigentliche Positionierschablone angefertigt. Häufig lässt die Passfähigkeit zu wünschen übrig, und die Kosten für die Bereitstellung sind enorm. Zum anderen können Implantatposition und -ausrichtung als Zahlenwerte auf Papier ausgedruckt werden. Diese Werte werden dann an einem separaten analogen Bohrtisch eingestellt und die Bohrungen vorgenommen (DE 196 29 708 A1). Hierbei kann eine neue Positionierschablone angefertigt werden oder aber die Scanschablone in eine Positionierschablone umgearbeitet werden.

Andere Lösungen präferieren die Überführung der Ergebnisse der Planung in ein Datenfile, dessen Informationen auf Papier ausgedruckt werden. Die ermittelten Werte der Planung werden danach an einem 3D-Tisch wiederum händisch eingegeben. Durch den Bruch in der Informationskette ist mit einem hohen zeitlichen Aufwand zu rechnen; der Ablauf ist zudem fehleranfällig. Die Kosten für die Investition des 3D-Tischs sind enorm; dieser ist allerdings für die Positionierschablonenfertigung unbedingt notwendig.

Bei einer durchgängig rechnergestützten Arbeitsweise werden gegenwärtig mehrere unterschiedliche Softwarelösungen (SKYplanX, Rhino, Delcam) nacheinander bis zur CNC-Herstellung der Positionierschablone genutzt. Dieser Prozess ist einem Zahntechniker sehr schwer vermittelbar, birgt enorme Fehlerquellen und setzt die Investition mehrerer preisintensiver unterschiedlicher Softwarelösungen voraus.

Aufgabe der vorliegenden Erfindung ist es, die zuvor aufgezeigten Nachteile bei der Herstellung einer Zahnimplantatstruktur zu überwinden und dadurch insbesondere die Herstellung von Zahnimplantatstrukturen zu vereinfachen.

Gelöst wird die Aufgabe gemäß der Erfindung mit einer Vorrichtung zur Verwendung in einem Verfahren zum Herstellen einer Zahnimplantatstruktur gemäß dem unabhängigen Anspruch 1 sowie einem Verfahren zur Herstellung einer Zahnimplantatstruktur gemäß dem unabhängigen Anspruch 17.

Bevorzugt sind die Haltemittel am oder im Referenzabschnitt vorgesehen und/oder weisen Aussparungen und/oder Löcher auf. Für eine eindeutige Zuordnung sollte bevorzugt für die Referenzelemente eine asymmetrische Anordnung vorgesehen sein. Des Weiteren sollten bevorzugt im Referenzabschnitt im Querschnitt kreisförmige Löcher oder Aussparungen zur, bevorzugt lösbar, arretierenden Aufnahme von als Kugeln ausgebildeten Referenzelementen vorgesehen sein.

Zweckmäßigerweise sollte der Referenzabschnitt im Wesentlichen als Platte ausgebildet sein. Bevorzugt können der Referenzabschnitt, der Schablonenabschnitt und die Haltemittel miteinander einstückig ausgebildet sein, alternativ ist es aber auch denkbar, den Referenzabschnitt lösbar am Schablonenabschnitt anzuordnen. Zur Befestigung mindestens eines Zahnersatzteils am Schablonenabschnitt sollten bevorzugt Befestigungsmittel, insbesondere Klebemittel verwendet werden.

Für eine eindeutige Differenzierung bei der Darstellung sollten bevorzugt der Referenzabschnitt, der Schablonenabschnitt und die Haltemittel im Wesentlichen nicht abbildbares Material und dagegen die Referenzelemente und das mindestens eine Zahnersatzteil im Wesentlichen abbildbares Material aufweisen, sodass bei einer Abbildung auf einem Bildschirm im Wesentlichen nur die Referenzelemente und das mindestens eine Zahnersatzteil erkennbar sind, auf welches es ja im Wesentlichen auch nur ankommt. Im Hinblick auf die Anatomie des Kiefers ist es von Vorteil, den Schablonenabschnitt im Wesentlichen mit einer entsprechend dem Kiefer gekrümmten Formgebung zu versehen. Für eine exakte Ausbildung von Bohrungen im Kiefer sollte bevorzugt der Schablonenabschnitt zur Einarbeitung mindestens eines als Durchgangsbohrung ausgebildeten Funktionselementes für die Aufnahme einer Bohrerführungshülse vorgesehen sein, die eine genaue Führung einer passenden Bohrspitze gestattet. Zur lösbaren Befestigung in einer definierten Anordnung am Kiefer des Patienten sollte bevorzugt der Schablonenabschnitt Befestigungsmittel, insbesondere Ankerpins, aufweisen.

Bei einer Weiterbildung ist die erste Verarbeitungseinrichtung ausgebildet, die Implantatdaten unter Berücksichtigung eines vorbestimmten Mindestabstandes zu einem benachbarten Hindernis wie insbesondere einem benachbarten Zahn, Implantat und/oder Nerv und/oder zu den Außenseiten des Kiefers zu erzeugen, so dass sie für die Anordnung eines Implantats nur einen festgelegten Raum definieren, der in einem Abstand zu einem benachbarten Hindernis bzw. den Außenseiten des Kiefers liegt, welcher mindestens dem vorbestimmten Mindestabstand entspricht.

Eine weitere bevorzugte Weiterbildung weist eine dritte Verarbeitungseinrichtung auf, die ausgebildet ist, die Implantatdaten unter Verwendung der Daten der von der zweiten Verarbeitungseinrichtung angegebenen passenden Implantatausführung entsprechend anzupassen.

Bei einer weiteren bevorzugten Weiterbildung ist die zweite Verarbeitungseinrichtung ausgebildet, als Ergebnis des Vergleiches aus der Vielzahl der unterschiedlichen Implantatausführungen eine Gruppe von passenden Implantatausführungen zur Auswahl durch den Anwender anzugeben.

Ferner wird beispielhaft ein Verfahren unter Verwendung der genannten Vorrichtung beschrieben, mit den Schritten,
A) mit einer Abtasteinrichtung Bilddaten vom Kiefer eines Patienten zu erzeugen,
B) in den Bilddaten aufgrund einer Festlegung der Position, der Orientierung und des Raumes für die Aufnahme eines Implantates Implantatdaten zu erzeugen, die Parameter für die Anordnung des Implantats wie insbesondere die Abmessungen des hierfür festgelegten Raumes angeben,
C) die Implantatdaten mit abgespeicherten Daten einer Vielzahl von unterschiedlichen Implantatausführungen zu vergleichen und als Ergebnis dieses Vergleiches mindestens eine passende Implantatausführung aus der Vielzahl der unterschiedlichen Implantatausführungen anzugeben, und
D) die Implantatdaten auf der Gundlage eines Referenzkoordinatensystems in Maschinendaten für eine im Zusammenhang mit dem Einsetzen des Implantates in den Kiefer zu verwendende Bearbeitungsmaschine zu transformieren,
wobei vor dem Schritt A der Schablonenabschnitt mit mindestens einem Zahnersatzteil versehen wird, zwischen dem Schritt A und dem Schritt B auf der Grundlage der in den Bilddaten enthaltenen Daten für die Referenzelemente ein Referenzkoordinatensystem definiert wird, und
nach dem Schritt D die Anordnung aus Schablonenabschnitt und Referenzabschnitt mit den Haltemitteln in eine Bearbeitungsmaschine eingespannt und mit Hilfe der Bearbeitungsmaschine unter Verwendung der Maschinendaten mindestens ein Funktionselement, bevorzugt eine Bohrung, zumindest in den Schablonenabschnitt der Vorrichtung eingearbeitet wird.

Vorzugsweise wird ein vorbestimmter Mindestabstand zu einem benachbarten Hindernis wie insbesondere einem benachbarten Zahn, Implantat und/oder Nerv festgelegt und werden im Schritt B die Implantatdaten unter Berücksichtigung dieses vorbestimmten Mindestabstandes erzeugt, sodass sie für die Anordnung eines Implantats nur einen solchen Raum definieren, der in einem Abstand zu einem benachbarten Hindernis liegt, welcher mindestens dem vorbestimmten Mindestabstand entspricht. Des Weiteren sollte bevorzugt ein vorbestimmter Mindestabstand zu den Außenseiten des Kiefers definiert und im Schritt B die Implantatdaten unter Berücksichtigung dieses vorbestimmten Mindestabstandes erzeugt werden, sodass sie für die Aufnahme eines Implantats nur einen solchen Raum definieren, der in einem Abstand zu den Außenseiten des Kiefers liegt, welcher mindestens dem vorbestimmten Mindestabstand entspricht.

Alternativ oder zusätzlich kann im Schritt C als Ergebnis des Vergleiches aus der Vielzahl der unterschiedlichen Implantatausführungen eine Gruppe von passenden Implantatausführungen zur Auswahl durch den Anwender angegeben werden. Zweckmäßigerweise sollten zwischen dem Schritt C und dem Schritt D die Implantatdaten unter Berücksichtigung der Daten der passenden Implantatausführung entsprechend angepasst werden.

Ferner ist bevorzugt ein weiterer Schritt vorzusehen, den Referenzabschnitt vom Schablonenabschnitt zu trennen.

Schließlich können die zuvor beschriebenen Verfahren bevorzugt in einem Computerprogramm implementiert werden.

Im Übrigen wird hinsichtlich der bevorzugten Ausführungen und Weiterbildungen der Erfindung auf die abhängigen Ansprüche verwiesen.

Die Erfindung zielt auf einen sicheren durchgängig digitalen Daten- und Informationsfluss von der Therapieplanung anhand von CT- und DVT-Schichtbildern bis zur CNC-gestützten Herstellung von Positionierschablonen für Operationen im Dentalbereich (z.B. Implantationen) ab. Die Erfindung ermöglicht eine automatische Transformation der therapierelevanten Planungskoordinaten (z.B. Implantat- und Bohrhülsenpositionen) in ein Fertigungskoordinatensystem insbesondere unter Nutzung von speziellen Referenzobjekten wie beispielsweise Referenzelemente in einem Referenzabschnitt, an dem ein Schablonenabschnitt angeordnet ist.

Grundlage für die Lösung der zuvor angegebenen Aufgaben bildet erfindungsgemäß ein Referenzabschnitt mit eindeutig definiertem Plattenkoordinatensystem, der während der Herstellung an einem Schablonenabschnitt unlösbar und fest in diese integriert wird. Der Vorteil dabei ist, dass der Referenzabschnitt bei der Befestigung gegenüber dem Schablonenabschnitt relativ frei positionierbar ist. Somit kann der Referenzabschnitt abweichend von der Zahnebene und unter Berücksichtigung der Gegebenheiten im Mundraum angebracht werden. Eine Beeinflussung des Transformationsergebnisses z.B. durch Artefakte in der volumentomografischen Aufnahme kann dadurch minimiert oder eliminiert werden. Der Referenzabschnitt wird bevorzugt aus einem nicht röntgenopaken festen Werkstoff gefertigt und dient somit gleichzeitig zur Stabilisierung des Schablonenabschnittes. Der Referenzabschnitt weist bevorzugt ein eindeutiges, unsymmetrisches Schema zur Aufnahme von (mindestens 3) Referenzelementen aus röntgenopakem Material (Keramik, Si3N4) auf. Die Informationen zu Anzahl, Durchmesser und Lage der Referenzelemente gegenüber dem Plattenkoordinatensystem sind bekannt und werden in einem Datenfile gespeichert.

Nachfolgend wird ein bevorzugtes Ausführungsbeispiel der Erfindung anhand der beiliegenden Zeichnungen näher erläutert. Es zeigen:
- Fig. 1: schematisch im Blockschaltbild eine Vorrichtung für eine durchgängig rechnergestützte Planung einer dentalen Versorgung gemäß einem bevorzugten Ausführungsbeispiel;
- Fig. 2: ein Ablaufdiagramm zur schematischen Darstellung der rechnergestützten Planung einer dentalen Versorgung mithilfe der in Fig. 1 dargestellten Vorrichtung;
- Fig. 3: eine Scanschablone mit daran befestigter Referenzplatte gemäß einem bevorzugten Ausführungsbeispiel der Erfindung in Anordnung auf dem Modell eines Unterkiefers (Fig. 3a) und in alleiniger Darstellung (Fig. 3b);
- Fig. 4: beispielhaft die Abbildung eines Kiefers mit einer Umrissplanung für ein neues Implantat sowie mit Darstellung eines Implantates;
- Fig. 5: die schematische Darstellung eines Bildstapels mit Plattenkoordinatensystem, das durch die Referenzplatte mithilfe der dort angeordneten Referenzkugeln definiert wird; und
- Fig.6: schematisch eine Draufsicht auf die als Positionierschablone zu verwendende Scanschablone mit dort eingebrachten Bohrhülsen.

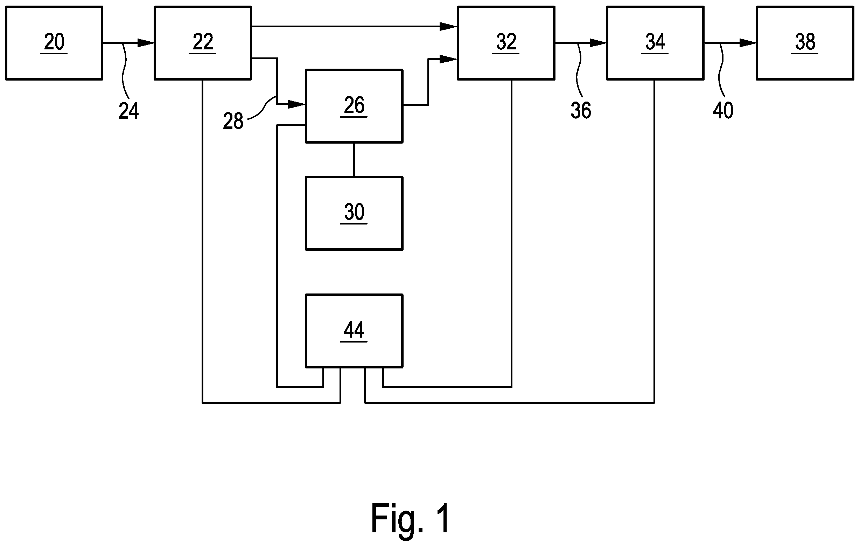

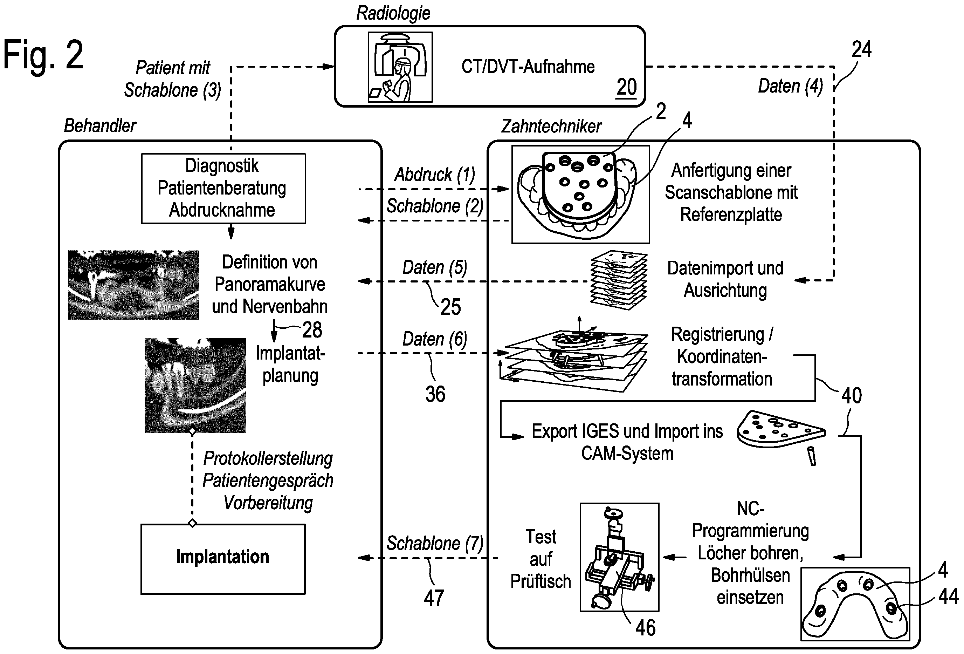

Die Vorrichtung und das Verfahren gemäß dem in den Figuren dargestellten Ausführungsbeispiel zielen auf einen sicheren durchgängig digitalen Daten- und Informationsfluss von der Therapieplanung anhand von CT- und DVT-Schichtbildern bis zur CNC-gestützten Herstellung von Positionierschablonen für Operationen im Dentalbereich (z.B. Implantationen) ab und ermöglichen eine automatische Transformation der therapierelevanten Planungskoordinaten (z.B. Implantat- und Bohrhülsenpositionen) in ein Fertigungskoordinatensystem unter Nutzung von speziellen Referenzobjekten (Referenzkugeln, Referenzplatte, Aufnahmevorrichtung), wie in einer Gesamtschau die Fig. 1, die beispielhaft schematisch eine Vorrichtung zeigt, und die Fig. 2, die beispielhaft ein Ablaufdiagramm zeigt, erkennen lassen.

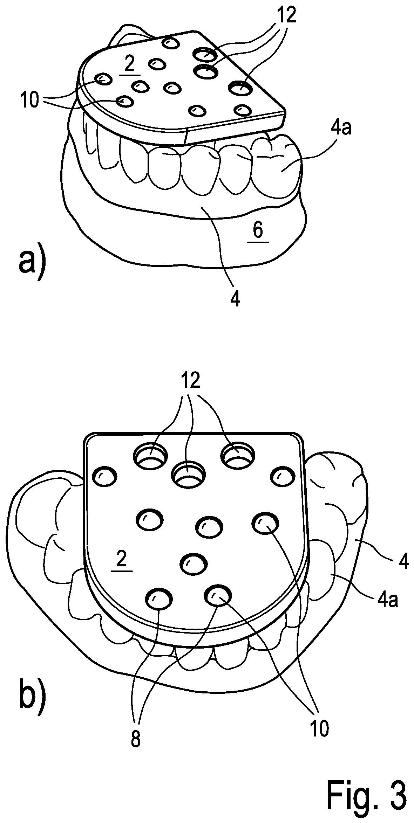

Grundlage hierfür bildet im dargestellten Ausführungsbeispiel eine Referenzplatte 2 mit eindeutig definiertem Plattenkoordinatensystem dar, die während der Herstellung einer Scanschablone 4 unlösbar und fest in diese integriert wird. Für die Anfertigung der Scanschabole 4 wird zunächst entweder von dem betreffenden Kiefer ein Abdruck erzeugt oder der betreffende Kiefer visuell abgetastet. Gemäß dem in den Figuren 2 und 3 dargestellten Ausführungsbeispiel ist die Scanschablone 4 für einen insgesamt zahnlosen Kiefer vorgesehen und somit über die gesamte Länge, die etwa auch der Länge des Kiefers entspricht, mit einer Anordnung von nachgebildeten Zahnersatzteilen 4a versehen. Im Hinblick auf die gekrümmte Form des Kiefers weist auch die Scanschablone 4 im dargestellten Ausführungsbeispiel im Wesentlichen eine entsprechend gekrümmte Formgebung auf. In einem solchen Fall ist es erforderlich, den gesamten Kiefer mit Implantaten zu versehen. Für den alternativen Fall, dass in dem Kiefer an einer bestimmten Stelle nur ein einziges Implantat oder nur einige wenige Implantate vorzusehen sind, ist die Scanschablone nur an dieser Stelle mit entsprechend nachgebildeten Zahnersatzteilen zu versehen.

Fig. 3 zeigt beispielhaft die Scanschablone 4 mit der daran befestigten Referenzplatte 2 in Anordnung auf dem Modell 6 eines Unterkiefers (Fig. 3a) sowie in alleiniger Darstellung (Fig. 3b). Der Vorteil dabei ist, dass die Referenzplatte 2 bei der Befestigung gegenüber der Scanschablone 4 relativ frei positionierbar ist. Somit kann die Referenzplatte 2 abweichend von der Zahnebene und unter Berücksichtigung der Gegebenheiten im Mundraum angebracht werden. Eine Beeinflussung des Transformationsergebnisses z.B. durch Artefakte in der volumentomografischen Aufnahme kann dadurch minimiert oder gar eliminiert werden. Die Referenzplatte 2 wird aus einem nicht röntgenopaken festen Werkstoff gefertigt und dient somit gleichzeitig zur Stabilisierung der Scanschablone 4. Die Referenzplatte weist ein eindeutiges, unsymmetrisches Schema von Bohrungen 8 zur Aufnahme von (mindestens 3) Referenzkugeln 10 aus röntgenopakem Material (Keramik, Si3N4) auf. Die Informationen zu Anzahl, Durchmesser und Lage der Referenzkugeln 10 gegenüber dem Plattenkoordinatensystem sind bekannt und werden in einem Datenfile gespeichert.

Die Referenzplatte 2 weist weiterhin eine asymmetrische Anordnung von (mindestens drei) Montagebohrungen 12 auf, welche eine formschlüssige, wiederholbare Einspannung in einer speziellen dafür angepassten in den Figuren nicht dargestellten Aufnahmevorrichtung ermöglicht. Ein versehentliches Verdrehen der Referenzplatte 2 beim Einlegen ist ausgeschlossen. Die Aufnahmevorrichtung lässt sich einmalig durch Einmessen anhand bekannter technologischer Basen (z.B. Ebenen) in Bezug zur Plattenaufnahme innerhalb einer Werkzeugmaschine positionieren (Fertigungskoordinatensystem).

Die Scanschablone 4 wird, wie bereits zuvor erwähnt, anhand eines Kieferabdrucks, dessen Modell in Fig. 3a beispielhaft dargestellt und mit dem Bezugszeichen "6" bezeichnet ist, und/oder eines digitalen Mundscans angefertigt. Nach Einarbeitung der Referenzplatte 2 mit den Referenzkugeln 10 in die Scanschablone 4 findet eine volumentomografische Aufnahme des betreffenden Kiefers des Patienten mit der Scanschablone 4 statt. Hierzu wird eine Abtasteinrichtung 20 verwendet, wie sie in den Figuren 1 und 2 schematisch angedeutet ist. Die Abtasteinrichtung 20 muss in der Lage sein, den Kiefer zu durchleuchten, und ist deshalb bevorzugt zur Erzeugung von CT-Aufnahmen ausgebildet. Somit werden mit Hilfe der Aufnahmeeinrichtung 20 bevorzugt sog. Schichtaufnahmen generiert, die in bestimmten Abständen voneinander jeweils eine Art Schnittbild darstellen und zusammengesetzt eine perspektivische Abbildung ergeben. Die von der Abtasteinrichtung 20 erzeugten Bilddaten werden in einer ersten Verarbeitungseinrichtung 22 verarbeitet, wobei die Übertragung der Bilddaten in den Figuren 1 und 2 schematisch durch einen Pfeil mit dem Bezugszeichen "24" angedeutet ist. Neben dem Import der Bilddatenbesteht eine Funktion der ersten Verarbeitungseinrichtung 22 darin, die Bilder in verschiedene Perspektiven zu verdrehen und/oder in ihrer Größe zu reduzieren und/oder die Bildanzahl eines Bildstapels zu verringern. Dabei wird in der ersten Verarbeitungseinrichtung 22 auf der Grundlage der in den Bilddaten enthaltenen Daten für die Referenzkugeln 10 ein Referenzkoordinatensystem definiert.

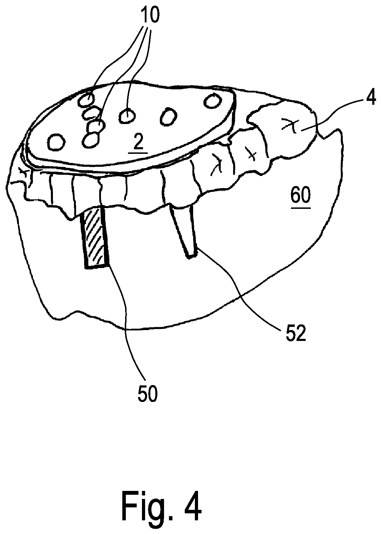

Eine weitere Aufgabe der ersten Verarbeitungseinrichtung 22 besteht darin, in den Bilddaten aufgrund einer Festlegung der Position, der Orientierung und des Raumes für die Aufnahme eines Implantates Implantatdaten zu erzeugen, die Parameter für die Anordnung des späteren Implantats wie insbesondere die Abmessungen des hierfür festgelegten Raumes angeben. Insbesondere bei Festlegung eines vorbestimmten Mindestabstandes zu einem benachbarten Hindernis wie insbesondere einem benachbarten Zahn, Implantat und/oder Nerv werden Implantatdaten unter Berücksichtigung dieses vorbestimmten Mindestabstandes erzeugt, sodass die Implantatdaten für die Anordnung eines Implantats nur einen solchen Raum definieren, der in einem Abstand von dem benachbarten Hindernis liegt, welcher mindestens dem vorbestimmten Mindestabstand entspricht. Des Weiteren sollte ein vorbestimmter Mindestabstand zu den Außenseiten des Kiefers definiert werden, sodass mithilfe der ersten Verarbeitungseinrichtung 22 die Implantatdaten auch unter Berücksichtigung dieses vorbestimmten Mindestabstandes erzeugt werden, indem für die Aufnahme eines Implantats nur ein solcher Raum zur Umrissplanung definiert wird, der in einem Abstand zu den Außenseiten des Kiefers liegt, welcher mindestens dem vorbestimmten Mindestabstand entspricht. Beispielhaft zeigt hierzu Fig. 4 eine aus den zuvor erwähnten Schichtbildern bzw. Bilddaten generierte dreidimensionale Abbildung auf einem Monitor, welche einen Kiefer 60 und eine darauf aufgesetzte Scanschablone 4 mit Referenzplatte 2 zeigt, wobei insbesondere auch deutlich die Referenzkugeln 10 zu erkennen sind. Des Weiteren ist in Fig. 4 mit dem Bezugszeichen "50" schematisch der zuvor bereits erwähnte festzulegende Raum erkennbar dargestellt, innerhalb dessen das spätere Implantat anzuordnen ist. Zur besseren Erkennbarkeit ist der Raum 56 schraffiert dargestellt. Des Weiteren lässt die Abbildung von Fig. 4 beispielhaft noch ein Implantat 52 erkennen, bei dem es sich beispielsweise um ein bereits vorhandenes Implantat handeln kann. In Fig. 2 ist diese interne Weiterverarbeitung der Implantatdaten schematisch durch den Pfeil 25 angedeutet.

Anschließend werden die so von der ersten Verarbeitungseinrichtung 22 verarbeiteten und erzeugten Implantatdaten in einer zweiten Verarbeitungseinrichtung 26 weiterverarbeitet, wobei die Übertragung der Implantatdaten in den Figuren 1 und 2 schematisch durch den Pfeil 28 angedeutet ist. Die Weiterverarbeitung der Implantatdaten in der zweiten Verarbeitungseinrichtung 26 besteht darin, die Implantatdaten mit in einer Datenbank 30 abgespeicherten Daten für eine Vielzahl von unterschiedlichen Implantatausführungen zu vergleichen und als Ergebnis dieses Vergleiches mindestens eine passende Implantatausführung oder auch eine Gruppe von passenden Implantatausführungen zur Auswahl durch den Anwender aus der Vielzahl der unterschiedlichen Implantatausführungen anzugeben.

In einer dritten Verarbeitungseinrichtung 32 (Fig. 1) werden die von der ersten Verarbeitungseinrichtung 22 ausgegebenen Implantatdaten unter Verwendung der Daten der von der zweiten Verarbeitungseinrichtung 26 angegebenen passenden Implantatausführung(en) entsprechend angepasst. Die ausgewählte passende Implantatausführung wird nun visualisiert und virtuell in dem besagten Raum 50 angeordnet, indem in die Bilddaten die Daten der ausgewählten passenden Implantatausführung entsprechend eingearbeitet werden. Dementsprechend kann es sich bei dem in Fig. 4 abgebildeten Implantat 52 alternativ auch um ein derartiges visuelles Implantat handeln, das dann als Vorlage für das spätere Einsetzen eines realen Implantates dient.

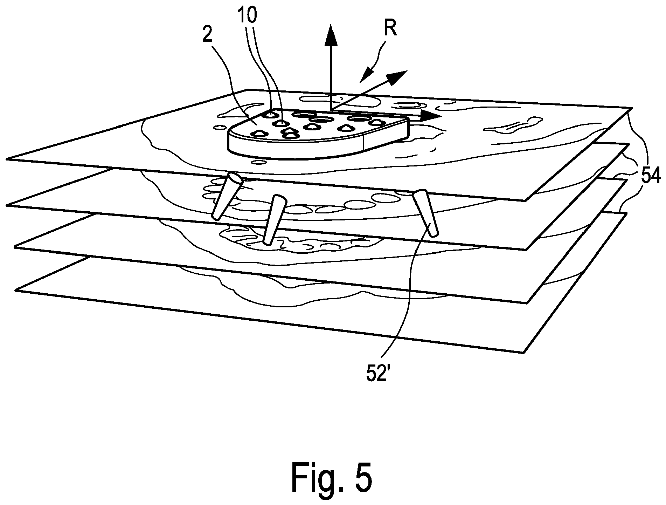

In Fig. 5 ist zur Veranschaulichung ein Bildstapel 54 dargestellt, der beispielhaft aus vier Schichtbildern besteht. Wie Fig. 5 des weiteren schematisch erkennen lässt, wird für die Bilddaten und die Implantatkoordinaten ein Referenzplattenkoordinatensystem verwendet, das durch die Referenzplatte 2 definiert ist. Die mit dem Bezugszeichen "52''' in Fig. 5 gekennzeichneten Elemente bilden geplante visualisierte Implantate, die in einer gewünschten Ausrichtung zur Referenzplatte 2 und somit auch in Bezug auf das Referenzplattenkoordinatensystem R orientiert sind.

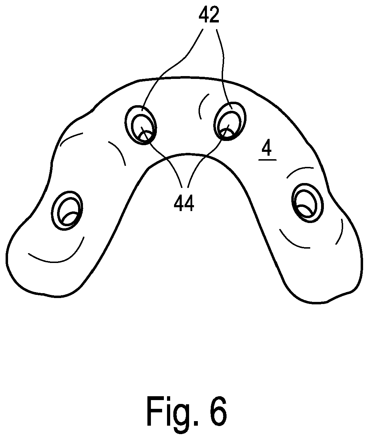

Die so von der dritten Verarbeitungseinrichtung 32 angepassten Implantatdaten werden dann in einer Transformationseinrichtung 34 auf der Grundlage des Referenzkoordinatensystems in Maschinendaten für eine im Zusammenhang mit dem Einsetzen des Implantates in den Kiefer zu verwendende Bearbeitungsmaschine 38 transformiert, die bevorzugt als CNC-Maschine ausgebildet ist. Hierzu wird die Anordnung aus Referenzplatte 2 und Scanschablone 4 mithilfe der Montagebohrungen 12 (Fig. 3) in die Bearbeitungsmaschine 38 eingespannt und mit deren Hilfe unter Verwendung der Maschinendaten von der Transformationseinrichtung 34 Funktionselemente, die im hier beschriebenen Ausführungsbeispiel gemäß Figur 6 als Bohrung 42 ausgebildet sind, in die Scanschablone 4 eingearbeitet. In die in der Scanschablone 4 eingearbeiteten Bohrungen 42 wird jeweils eine ebenfalls in Figur 6 schematisch dargestellte Bohrhülse 44 eingesetzt, die zur Führung einer in den Figuren nicht gezeigten Bohrspitze dient. Die Übertragung der Daten zur Transformationseinrichtung 34 ist in Figuren 1 und 2 schematisch durch den Pfeil 36 und von der Transformationseinrichtung 34 zur Bearbeitungsmaschine 38 schematisch durch den Pfeil 40 angedeutet.

Nach einem Test auf einem Prüftisch 46 wie in Fig. 2 schematisch angedeutet ist, wird die Referenzplatte 2 von der Scanschablone 4 getrennt.

Anschließend wird in den Kiefer des Patienten an der gewünschten Stelle eine Bohrung eingebracht, in der dann später das Implantat angeordnet wird. Hierzu wird die Scanschablone 4 auf den betreffenden Kiefer aufgesetzt und übernimmt nun die Aufgabe einer Positionierschablone, indem die in die Bohrung 42 in der Scanschablone 4 eingesetzte Bohrhülse 44 (Figur 6) zur exakten Führung einer passenden Bohrspitze eines ebenfalls in den Figuren nicht dargestellten Bohrwerkzeuges dient, wodurch die Einbringung der Bohrung exakt in der gewünschten Ausrichtung und an der gewünschten Stelle im Kiefer gewährleistet wird. Dieser im vorliegend beschriebenen Ausführungsbeispiel letzte Verfahrensschritt ist in Fig. 2 schematisch durch den Pfeil 47 angedeutet.

Um zumindest die zuvor beschriebenen relevanten Datenverarbeitungsschritte visuell zu überwachen und auch beeinflussen zu können, ist ein Terminal 44 mit Monitor und Tastatur vorgesehen, das insbesondere an die Verarbeitungseinrichtungen 22, 26 und 32 und die Transformationseinrichtung 34 angeschlossen ist, wobei sich mit dem Monitor u.a.die in Figur 4 schematisch gezeigte Abbildung darstellen lässt.

Ferner sei an dieser Stelle noch ergänzend angemerkt, dass insbesondere die Verarbeitungseinrichtungen 22, 26, 32, die Datenbank 30, die Transformationseinrichtung 34 und das Eingabeterminal 44 zumindest teilweise oder auch vollständig Bestandteile einer elektronischen Datenverarbeitungsanlage sein können.

Das zuvor beschriebene Verfahren zielt auf einen sicheren durchgängig digitalen Daten- und Informationsfluss von der Therapieplanung anhand von CT- und DVT-Schichtbildern bis zur CNC-gestützten Herstellung von Positionierschablonen für Operationen im Dentalbereich (z.B. Implantationen) ab und ermöglicht eine automatische Transformation des therapierelevanten Planungskoordinatensystems (z.B. Implantat- und Bohrhülsenpositionen) in ein Fertigungskoordinatensystem unter Nutzung von speziellen Referenzobjekten (Referenzkugeln 10, Referenzplatte 2, Aufnahmevorrichtung).

Eine Grundlage stellt die Referenzplatte 2 mit eindeutig definiertem Plattenkoordinatensystem R (Figur 5) dar, die während der Herstellung der Scanschablone 4 unlösbar und fest in diese integriert wird. Der Vorteil dabei ist, dass die Referenzplatte 2 bei der Befestigung gegenüber der Scanschablone 4 relativ frei positionierbar ist. Somit kann die Referenzplatte 2 abweichend von der Zahnebene und unter Berücksichtigung der Gegebenheiten im Mundraum angebracht werden. Eine Beeinflussung des Transformationsergebnisses z.B. durch Artefakte in der volumentomografischen Aufnahme kann dadurch minimiert oder eliminiert werden. Die Referenzplatte 2 wird aus einem nicht röntgenopaken festen Werkstoff gefertigt und dient somit gleichzeitig zur Stabilisierung der Scanschablone 4. Die Referenzplatte 2 weist ein eindeutiges, unsymmetrisches Schema von Bohrungen 8 zur Aufnahme von (mindestens 3) Referenzkugeln 10 aus röntgenopakem Material (Keramik, Si3N4) auf. Die Informationen zu Anzahl, Durchmesser und Lage der Kugeln 10 gegenüber dem Plattenkoordinatensystem R (Figur 5) sind bekannt und werden in einem Datenfile gespeichert.

Durch intelligente Kombination geeigneter Algorithmen werden die Positionen der Mittelpunkte der Kugeln 10 in der Referenzplatte 2 in Bezug auf das Referenzplattenkoordinatensystem R das ja auch das Koordinatensystem für den erzeugten Bildstapel 54 gemäß Figur 5 definiert, bestimmt. Dafür erfolgt eine erste Detektion der Kugelregionen innerhalb der einzelnen 2D-Schichtbilder durch die Bestimmung der Schnittkreise im schwellwertbasierten Kantenbild. Anschließend werden im dreidimensionalen Umfeld der gefundenen Regionen voxelbasiert die Kugelgeometrie und deren Mittelpunkt gesucht.

Aus dem Datenfile sind die Positionen der Kugeln 10 in der Referenzplatte 2 gegenüber dem Referenzplattenkoordinatensystem R (Figur 5) bekannt. Die Zuordnung der gefundenen Kugelmittelpunkte zu den bekannten Referenzkugelpositionen erfolgt aufgrund des oben genannten eindeutigen, unsymmetrischen Schemas der Bohrungen 8 in der Referenzplatte 2. Über die Korrespondenz der Mittelpunkte lässt sich eine Gesamttransformationsmatrix berechnen.

Wie bereits weiter oben angesprochen und insbesondere in Figur 3b erkennbar, zeichnet sich die Referenzplatte 2 weiterhin durch eine asymmetrische Anordnung der Montagebohrungen 12 aus, welche eine formschlüssige, wiederholbare Einspannung in einer speziellen dafür angepassten Aufnahmevorrichtung ermöglicht. Ein versehentliches Verdrehen der Platte 2 beim Einlegen ist ausgeschlossen. Die Aufnahmevorrichtung lässt sich einmalig durch Einmessen anhand bekannter technologischer Basen (z.B. Ebenen) in Bezug zur Plattenaufnahme innerhalb einer Werkzeugmaschine positionieren (Fertigungskoordinatensystem).

Die Anwendung im Gesamtkontext gestaltet sich wie folgt:
1. Anfertigung einer patientenspezifischen Scanschablone 4 anhand eines Kieferabdrucks 6 und/oder digitalen Mundscans;
2. Einarbeitung der Referenzplatte 2 mit Referenzkugeln 10 in die Scanschablone 4 (Fig.3);
3. Volumentomografische Aufnahme des Patienten mit Scanschablone 4;
4. Planung der medizinischen Versorgung (z.B. Implantate) im 3D-Volumendatensatz mit Hilfe einer Software in Abhängigkeit vom Koordinatensystem des Bildstapels 54 (Fig. 5);
5. Kugelfindung über oben beschriebenen Algorithmus im Bildstapel 54 und Berechnung der Mittelpunkte der Kugeln 10 in der Referenzplatte 2;
6. Anpassung an das Referenzplattenkoordinatensystem R (Fig. 5) über oben beschriebenen Ablauf (Fig. 5);
7. Export der transformierten relevanten Geometrie in einem (neutralen) Datenformat als Datei;
8. Import der Datei im CAM-System, Ermittlung der Bearbeitungsreihenfolge und Generierung eines CNC-Programms;
9. Einspannen der Scanschablone 4 in die oben beschriebene Aufnahmevorrichtung der NC-Maschine und Einbringen der Funktionselemente, die im dargestellten Ausführungsbeispiel Bohrungen 42 (Fig. 6) bilden, laut CAM-Planung, wodurch die Scanschablone 4 somit zur Positionierschablone umgearbeitet wird;
10. Nutzung der erstellten Positionierschablone 4 mit integrierten Funktionselementen (Bohrungen 42) zur dentalen Versorgung des Patienten.

Ein Aspekt ist die automatische Transformation von Koordinaten geplanter Funktionselemente vom Planungs- in das Fertigungskoordinatensystem unter Nutzung der Referenzplatte 2 mit definiert angeordneten Referenzkugeln 10, einem Algorithmus zur automatischen Kugeldetektion und Berechnung der Transformation sowie einer eindeutigen Aufnahmevorrichtung für die NC-Fertigung. Damit wird eine durchgängig digitale, dentale Versorgungsplanung und die Unterstützung der Versorgung durch eine CNC-gefertigte Positionierschablone mit integrierten Funktionselementen ermöglicht.

Als Vorteile ergeben sich:
- Automatische Transformation der Koordinaten ohne Zutun des Anwenders;
- Nutzung standardisierter Austauschformate zur Überführung der Daten von der Planung zur Fertigung;
- Kombination von Planung und Fertigung zu einem gesamteinheitlichen digitalen Workflow (durchgängige Technologie);
- Die Nutzung jeder mehrachsigen NC- oder CNC-Fräsmaschine zur Fertigung ist möglich aufgrund der Anwendung von CAM-Technologien und der Bereitstellung einer eindeutigen Aufnahmevorrichtung (kein Verdrehen möglich durch asymmetrische Stiftaufnahme);
- Freie Positioniermöglichkeit der Referenzplatte 2 gegenüber der Zahnebene;
- Zusätzliche Stabilisierung der Scan- bzw. Positionierschablone 4;
- Die Kugeln 10 werden in die Referenzplatte 2 eingepresst und können nach Sterilisierung erneut verwendet werden;
- Einfacher Aufbau der Platte 2 erlaubt kostengünstige Fertigungsmöglichkeit (generativ oder durch Fräsen);
- Der Einsatz standardisierter Kugeln 10, die nach vorgegebenen Toleranzklassen gefertigt wurden, ermöglicht gleichbleibende Qualität und Genauigkeit bei geringen Kosten.

Als Besonderheiten der dabei verwendeten Scan-Schablone 4 mit der Referenzplatte 2 ist folgendes festzuhalten:
- Referenzplatte 2 mit eindeutig definiertem Plattenkoordinatensystem R;
- Referenzplatte 2 weist ein eindeutiges, unsymmetrisches Schema von Bohrungen 8 zur Aufnahme von (bevorzugt mindestens 3) Referenzkugeln 10 aus röntgenopakem Material (bevorzugt Keramik, Si3N4) auf;
- Referenzplatte 2 zeichnet sich durch eine asymmetrische Anordnung der Montagebohrungen 12 aus, welche eine formschlüssige, wiederholbare Einspannung in einer speziellen dafür angepassten Aufnahmevorrichtung ermöglicht;
- Referenzplatte 2 wird aus einem nicht röntgenopaken festen Werkstoff gefertigt;
- Freie Positioniermöglichkeit der Referenzplatte 2 gegenüber der Zahnebene;
- Zusätzliche Stabilisierung der Scan- bzw. Positionierschablone 4;
- Die Kugeln 10 werden in die Referenzplatte 2 eingepresst und können nach Sterilisierung erneut verwendet werden;
- Einfacher Aufbau der Platte 2 erlaubt kostengünstige Fertigungsmöglichkeit (bevorzugt generativ oder durch Fräsen);
- Der Einsatz standardisierter Kugeln 10, die nach vorgegebenen Toleranzklassen gefertigt wurden, ermöglicht gleichbleibende Qualität und Genauigkeit bei geringen Kosten.

Durch intelligente Kombination geeigneter Algorithmen werden die Positionen der Kugelmittelpunkte gegenüber dem Referenzplattenkoordinatensystem R gemäß Fig. 5 bestimmt, an das das Koordinatensystem des Bildstapels 54 entsprechend angepasst ist. Dafür erfolgt eine erste Detektion der Kugelregionen innerhalb der gemeinsam den Bildstapel 54 bildenden einzelnen 2D-Schichtbilder (Fig. 5) durch die Bestimmung der Schnittkreise im schwellwertbasierten Kantenbild. Anschließend werden im dreidimensionalen Umfeld der gefundenen Regionen voxelbasiert (d.h. auf den Bilddaten, nicht auf Polygondaten wie z.B. STL) die Kugelgeometrie und deren Mittelpunkt gesucht. Es sind die Kugelpositionen gegenüber dem Referenzplattenkoordinatensystem R (Figur 5) bekannt. Die Zuordnung der gefundenen Kugelmittelpunkte zu den bekannten Referenzkugelpositionen erfolgt aufgrund des eindeutigen, unsymmetrischen Schemas der Bohrungen 8 der Referenzplatte 2. Über die Korrespondenz der Mittelpunkte der Kugeln 10 in der Referenzplatte 2 lässt sich eine Gesamttransformationsmatrix berechnen.

## Patentansprüche

1. Vorrichtung zur Verwendung in einem Verfahren zum Herstellen einer Zahnimplantatstruktur, mit
einer Abtasteinrichtung (20), die vorgesehen ist, den Kiefer eines Patienten abzutasten und daraus entsprechende Bilddaten zu erzeugen,
einer ersten Verarbeitungseinrichtung (22), die ausgebildet ist, in den Bilddaten zur Festlegung einer Position, einer Orientierung und eines Raumes (50) für die Anordnung eines Implantates Implantatdaten zu erzeugen, die Parameter für die Anordnung des Implantats wie insbesondere die Abmessungen des hierfür festgelegten Raumes (50) angeben, einer zweiten Verarbeitungseinrichtung (26), die ausgebildet ist, die Implantatdaten mit abgespeicherten Daten einer Vielzahl von unterschiedlichen Implantatausführungen zu vergleichen und als Ergebnis dieses Vergleiches mindestens eine passende Implantatausführung aus der Vielzahl der unterschiedlichen Implantatausführungen anzugeben, und
einer Transformationseinrichtung (34), die ausgebildet ist, die Implantatdaten auf der Grundlage eines Referenzkoordinatensystems (R) in Maschinendaten für eine im Zusammenhang mit dem Einsetzen des Implantates in den Kiefer zu verwendende Bearbeitungsmaschine (38) zu transformieren,
**gekennzeichnet durch**
einen Referenzabschnitt (2), an oder in dem Referenzelemente (10) zur Definition eines Referenzkoordinatensystems (R) anzuordnen sind,
einen Schablonenabschnitt (4), der zur Aufnahme des Referenzabschnittes (2) in einer definierten Anordnung, zur lösbaren Befestigung am Kiefer eines Patienten und zur Aufnahme oder Ausbildung mindestens eines Zahnersatzteiles vorgesehen ist, und
Haltemittel (12), die in einer definierten Position in Bezug auf die Referenzelemente (10) angeordnet und für ein lösbares Einspannen der Anordnung aus Schablonenabschnitt (4) und Referenzabschnitt (2) in der Bearbeitungsmaschine (38), insbesondere einer CNC-Fräsmaschine, zum Einarbeiten mindestens eines Funktionselementes (42), bevorzugt einer Bohrung, zumindest in den Schablonenabschnitt (4) ausgebildet sind.

2. Vorrichtung nach Anspruch 1, bei welcher die erste Verarbeitungseinrichtung (22) ausgebildet ist, die Implantatdaten unter Berücksichtigung eines vorbestimmten Mindestabstandes zu einem benachbarten Hindernis wie insbesondere einem benachbarten Zahn, Implantat und/oder Nerv und/oder zu den Außenseiten des Kiefers zu erzeugen, so dass sie für die Anordnung eines Implantats nur einen festgelegten Raum (50) definieren, der in einem Abstand zu einem benachbarten Hindernis bzw. den Außenseiten des Kiefers liegt, welcher mindestens dem vorbestimmten Mindestabstand entspricht.

3. Vorrichtung nach mindestens einem der vorangegangen Ansprüche, mit einer dritten Verarbeitungseinrichtung (32), die ausgebildet ist, die Implantatdaten unter Verwendung der Daten der von der zweiten Verarbeitungseinrichtung (26) angegebenen passenden Implantatausführung entsprechend anzupassen.

4. Vorrichtung nach mindestens einem der vorangegangenen Ansprüche, bei welcher die zweite Verarbeitungseinrichtung (26) ausgebildet ist, als Ergebnis des Vergleiches aus der Vielzahl der unterschiedlichen Implantatausführungen eine Gruppe von passenden Implantatausführungen zur Auswahl durch den Anwender anzugeben.

5. Vorrichtung nach mindestens einem der vorangegangenen Ansprüche, bei welcher die Haltemittel (12) am oder im Referenzabschnitt (2) vorgesehen sind und/oder Aussparungen und/oder Löcher aufweisen.

6. Vorrichtung nach mindestens einem der vorangegangenen Ansprüche, bei welcher für die Referenzelemente (10) eine asymmetrische Anordnung vorgesehen ist.

7. Vorrichtung nach mindestens einem der vorangegangenen Ansprüche, bei welcher im Referenzabschnitt (2) im Querschnitt kreisförmige Löcher oder Aussparungen (8) zur, bevorzugt lösbar, arretierenden Aufnahme von als Kugeln ausgebildeten Referenzelementen (10) vorgesehen sind.

8. Vorrichtung nach mindestens einem der vorangegangenen Ansprüche, bei welcher der Referenzabschnitt (2) im Wesentlichen als Platte ausgebildet ist.

9. Vorrichtung nach mindestens einem der vorangegangenen Ansprüche, bei welcher der Referenzabschnitt (2), der Schablonenabschnitt (4) und die Haltemittel (12) miteinander einstückig ausgebildet sind.

10. Vorrichtung nach mindestens einem der Ansprüche 1 bis 8, bei welcher der Referenzabschnitt (2) lösbar am Schablonenabschnitt (4) angeordnet ist.

11. Vorrichtung nach mindestens einem der vorangegangenen Ansprüche, mit Befestigungsmitteln, insbesondere Klebemitteln, zur Befestigung mindestens eines Zahnersatzteils (4a) am Schablonenabschnitt (4).

12. Vorrichtung nach mindestens einem der vorangegangenen Ansprüche, bei welcher der Referenzabschnitt (2), der Schablonenabschnitt (4) und die Haltemittel (12) im Wesentlichen nicht abbildbares Material und die Referenzelemente (10) und das mindestens eine Zahnersatzteil (4a) im Wesentlichen abbildbares Material aufweisen.

13. Vorrichtung nach mindestens einem der vorangegangenen Ansprüche, bei welcher der Schablonenabschnitt (4) im Wesentlichen eine entsprechend dem Kiefer (6) gekrümmte Formgebung aufweist.

14. Vorrichtung nach mindestens einem der vorangegangenen Ansprüche, bei welcher der Schablonenabschnitt (4) zur Einarbeitung mindestens eines als Durchgangsbohrung ausgebildeten Funktionselementes (42) für die Aufnahme einer Bohrerführungshülse (44) vorgesehen ist.

15. Vorrichtung nach mindestens einem der vorangegangenen Ansprüche, **dadurch gekennzeichnet, dass** der Schablonenabschnitt (4) Befestigungsmittel, insbesondere Ankerpins, zur lösbaren Befestigung in einer definierten Anordnung am Kiefer des Patienten aufweist.

16. Verfahren zur Herstellung einer Zahnimplantatstruktur unter Verwendung einer Vorrichtung nach mindestens einem der vorangegangenen Ansprüche, mit den Schritten,
A) den Schablonenabschnitt (4) mit mindestens einem Zahnersatzteil zu versehen,
B) mit der Abtasteinrichtung (20) Bilddaten vom Kiefer eines Patienten mit dem daran befestigten Schablonenabschnitt (4) zu erzeugen,
C) auf der Grundlage der in den Bilddaten enthaltenen Daten für die Referenzelemente (10) ein Referenzkoordinatensystem (R) zu definieren,
D) mittels der ersten Verarbeitungseinrichtung (22) in den Bilddaten zur Festlegung der Position, der Orientierung und des Raumes (50) für die Aufnahme eines Implantates Implantatdaten zu erzeugen, die Parameter für die Anordnung des Implantats wie insbesondere die Abmessungen des hierfür festgelegten Raumes (50) angeben,
E) die Implantatdaten mittels der zweiten Verarbeitungseinrichtung (26) mit abgespeicherten Daten einer Vielzahl von unterschiedlichen Implantatausführungen zu vergleichen und als Ergebnis dieses Vergleiches mindestens eine passende Implantatausführung aus der Vielzahl der unterschiedlichen Implantatausführungen anzugeben,
F) die Implantatdaten mittels der Transformationseinrichtung (34) auf der Grundlage eines Referenzkoordinatensystems (R) in Maschinendaten für eine im Zusammenhang mit dem Einsetzen des Implantates in den Kiefer zu verwendende Bearbeitungsmaschine (38) zu transformieren,
G) die Anordnung aus Schablonenabschnitt (2) und Referenzabschnitt (4) mit den Haltemitteln (12) in eine Bearbeitungsmaschine (38) einzuspannen und
H) mit Hilfe der Bearbeitungsmaschine (38) unter Verwendung der Maschinendaten das mindestens eine Funktionselement (42), bevorzugt eine Bohrung, zumindest in den Schablonenabschnitt (4) einzuarbeiten.

17. Verfahren nach Anspruch 16, bei welchem im Schritt D die Implantatdaten unter Berücksichtigung eines vorbestimmten Mindestabstandes zu einem benachbarten Hindernis wie insbesondere einem benachbarten Zahn, Implantat und/oder Nerv und/oder zu den Außenseiten des Kiefers erzeugt werden, so dass sie für die Anordnung eines Implantats nur einen solchen Raum (50) definieren, der in einem Abstand zu einem benachbarten Hindernis bzw. den Außenseiten des Kiefers liegt, welcher mindestens dem vorbestimmten Mindestabstand entspricht.

18. Verfahren nach Anspruch 16 oder 17, bei welchem im Schritt E als Ergebnis des Vergleiches aus der Vielzahl der unterschiedlichen Implantatausführungen eine Gruppe von passenden Implantatausführungen zur Auswahl durch den Anwender angegeben wird.

19. Verfahren nach mindestens einem der Ansprüche 16 bis 18, bei welchem zwischen dem Schritt E und dem Schritt F die Implantatdaten unter Berücksichtigung der Daten der passenden Implantatausführung entsprechend angepasst werden.

## Claims

1. Device for use in a method for producing a tooth implant structure, comprising
a scanning device (20), which is provided for scanning the jaw of a patient and producing corresponding image data from the latter,
a first processing device (22) which is designed to produce implant data in the image data for establishing a position, an orientation and a space (50) for the arrangement of an implant, to specify the parameters for the arrangement of the implant such as in particular the dimensions of the space (50) established for this,
a second processing device (26) which is designed to compare the implant data with stored data of a plurality of different implant designs, and as a result of this comparison to specify at least one suitable implant design from the plurality of different implant designs, and
a transformation device (34) which is designed to transform the implant data on the basis of a reference coordinate system (R) into machine data for a processing machine (38) to be used in connection with inserting the implant into the jaw,
**characterised by**
a reference section (2), on or in which reference elements (10) are arranged for defining a reference coordinate system (R),
a template section (4), which is provided for mounting the reference section (2) in a defined arrangement for fixing detachably to the jaw of a patient and for mounting or forming at least one denture part, and
holding means (12) which are arranged in a defined position in relation to the reference elements (10) and are designed for releasably clamping the arrangement of the template section (4) and reference section (2) in the processing machine (38), in particular a CNC milling machine, for incorporating at least one functional element (42), preferably a bore, at least in the template section (4).

2. Device according to claim 1, wherein the first processing device (22) is designed to produce the implant data taking into account a predetermined minimum distance from an adjacent obstacle, such as in particular an adjacent tooth, implant and/or nerve and/or to the outsides of the jaw, so that for the arrangement of an implant they only define a specified space (50), which is located at a distance from an adjacent obstacle or from the outsides of the jaw which corresponds at least to the predefined minimum distance.

3. Device according to at least one of the preceding claims, with a third processing device (32) which is designed to adjust the implant data accordingly by using the data of the suitable implant design specified by the second processing device (26).

4. Device according to at least one of the preceding claims, wherein the second processing device (26) is designed, as a result of the comparison of the plurality of different implant designs to specify a group of suitable implant designs for selection by the user.

5. Device according to at least one of the preceding claims, wherein the holding means (12) are provided on or in the reference section (2) and/or have recesses and/or holes.

6. Device according to at least one of the preceding claims, wherein an asymmetrical arrangement is provided for the reference elements (10).

7. Device according to at least one of the preceding claims, wherein in the reference section (2) in cross-section circular holes or recesses (8) are provided for fixing, preferably detachably, reference elements (10) designed in the form of balls.

8. Device according to at least one of the preceding claims, wherein the reference section (2) is designed essentially as a plate.

9. Device according to at least one of the preceding claims, wherein the reference section (2), the template section (4) and the holding means (12) are formed in one piece with one another.

10. Device according to at least one of claims 1 to 8, wherein the reference section (2) is arranged detachably on the template section (4).

11. Device according to at least one of the preceding claims, comprising fastening means, in particular adhesive means, for securing at least one denture part (4b) onto the template section (4).

12. Device according to at least one of the preceding claims, wherein the reference section (2), the template section (4) and the holding means (12) comprise essentially non-presentable material and the reference elements (10) and the at least one denture part (4a) comprise essentially presentable material.

13. Device according to at least one of the preceding claims, wherein the template section (4) has essentially a curved form corresponding to the jaw (6).

14. Device according to at least one of the preceding claims, wherein the template section (4) is provided for introducing at least one functional element (42) in the form of a through-bore for mounting a bore guiding sleeve (44).

15. Device according to at least one of the preceding claims, **characterised in that** the template section (4) comprises fastening means, in particular anchor pins, for fastening detachably in a defined arrangement on the jaw of the patient.

16. Method for producing a tooth implant structure by using a device according to at least one of the preceding claims, comprising the steps:
A) providing the template section (4) with at least one denture part,
B) producing with the scanning device (20) image data of the jaw of a patient with the template section (4) fixed onto the latter,
C) on the basis of the data contained in the image data defining a reference coordinate system (R) for the reference elements (10),
D) by means of the first processing device (22) producing implant data in the image data for establishing the positon, the orientation and the space (50) for mounting an implant which specify parameters for the arrangement of the implant such as in particular the dimensions of the space (50) defined therefor,
E) comparing the implant data by mean of the second processing device (26) with saved data on a plurality of different implant designs and specifying as a result of this comparison a least one suitable implant design from the plurality of different implant designs,
F) transforming the implant data by means of the transformation device (34) on the basis of a reference coordinate system (R) into machine data for a processing machine (38) to be used in connection with inserting the implant into the jaw,
G) clamping the arrangement of template section (2) and reference section (4) to the holding means (12) into a processing machine and
H) by means of the processing machine (38) by using the machine data incorporating the at least one functional element (42), preferably a bore, at least into the template section (4).

17. Method according to claim 16, wherein in step D the implant data are produced taking into account a predefined minimum distance from an adjacent obstacle such as in particular an adjacent tooth, implant and/or nerve and/or to the outsides of the jaw so that they only define for the arrangement of an implant such as space (50) which is at a distance from an adjacent obstacle or the outsides of the jaw which corresponds at least to the predefined minimum distance.

18. Method according to claim 16 or 17, wherein in step E as a result of the comparison of the plurality of different implant designs a group of suitable implant designs is indicated for selection by the user.

19. Method according to a least one of claims 16 to 18, wherein between step E and step F the implant data are adjusted accordingly taking into account the data of the suitable implant design.

## Revendications

1. Dispositif destiné à être utilisé dans un procédé pour fabriquer une structure d'implant dentaire, avec
un système de balayage (20), qui est prévu pour balayer la mâchoire d'un patient et pour générer sur cette base des données d'image correspondantes,
un premier système de traitement (22), qui est réalisé pour produire sur les données d'image pour arrêter une position, une orientation et un espace (50) pour l'agencement de l'implant, des données d'implant, qui indiquent des paramètres pour l'agencement de l'implant tels qu'en particulier les dimensions de l'espace (50) arrêté à cet effet,
un deuxième système de traitement (26), qui est réalisé pour comparer les données d'implant à des données sauvegardées d'une pluralité de différentes réalisations d'implant et pour indiquer en tant que résultat de ladite comparaison au moins une réalisation d'implant appropriée issue de la pluralité des différentes réalisations d'implant, et
un système de transformation (34), qui est réalisé pour transformer les données d'implant sur la base d'un système de coordonnées de référence (R) en des données de machine pour une machine d'usinage (38) à utiliser en lien avec l'insertion de l'implant dans la mâchoire,
**caractérisé par**
une section de référence (2), au niveau de ou dans laquelle des éléments de référence (10) sont à agencer pour définir un système de coordonnées de référence (R),
une section de gabarit (4), qui est prévue pour loger la section de référence (2) selon un agencement défini, destinée à être fixée de manière amovible au niveau de la mâchoire d'un patient et à recevoir ou réaliser au moins une partie de prothèse dentaire, et
des moyens de maintien (12), qui sont disposés dans une position définie par rapport aux éléments de référence (10) et sont réalisés pour enserrer de manière amovible l'ensemble composé de la section de gabarit (4) et de la section de référence (2) dans la machine d'usinage (38), en particulier une fraiseuse CNC, pour pratiquer au moins un élément fonctionnel (42), de manière préférée, un alésage, au moins dans la section de gabarit (4).

2. Dispositif selon la revendication 1, dans lequel le premier système de traitement (22) est réalisé pour produire les données d'implant en tenant compte d'une distance minimale prédéfinie par rapport à un obstacle adjacent tel qu'une dent, un implant et/ou un nerf adjacent(e) et/ou par rapport aux côtés extérieurs de la mâchoire de sorte qu'elles définissent pour l'agencement d'un implant seulement un espace (50) arrêté, qui se situe à une distance par rapport à un obstacle adjacent ou aux côtés extérieurs de la mâchoire, laquelle correspond au moins à la distance minimale prédéfinie.

3. Dispositif selon au moins l'une quelconque des revendications précédentes, avec un troisième système de traitement (32), qui est réalisé pour adapter de manière correspondante les données d'implant en utilisant les données de la réalisation d'implant appropriée indiquée par le deuxième système de traitement (26).

4. Dispositif selon au moins l'une quelconque des revendications précédentes, où le deuxième système de traitement (26) est réalisé pour indiquer en tant que résultat de la comparaison parmi la pluralité des différentes réalisations d'implant un groupe de réalisations d'implant appropriées aux fins de la sélection par l'utilisateur.

5. Dispositif selon au moins l'une quelconque des revendications précédentes, où les moyens de maintien (12) sont prévus au niveau de ou dans la section de référence (2) et/ou présentent des évidements et/ou des trous.

6. Dispositif selon au moins l'une quelconque des revendications précédentes, où un agencement asymétrique est prévu pour les éléments de référence (10) .

7. Dispositif selon au moins l'une quelconque des revendications précédentes, où des trous ou des évidements (8) de forme circulaire dans la section transversale sont prévus pour loger par blocage, de manière préférée de manière amovible, des éléments de référence (10) réalisés en tant que billes dans la section de référence (2).

8. Dispositif selon au moins l'une quelconque des revendications précédentes, où la section de référence (2) est réalisée sensiblement en tant qu'une plaque.

9. Dispositif selon au moins l'une quelconque des revendications précédentes, où la section de référence (2), la section de gabarit (4) et les moyens de maintien (12) sont réalisés d'un seul tenant les uns avec les autres.

10. Dispositif selon au moins l'une quelconque des revendications 1 à 8, où la section de référence (2) est disposée de manière amovible au niveau de la section de gabarit (4).

11. Dispositif selon au moins l'une quelconque des revendications précédentes, avec dès moyens de fixation, en particulier des moyens de collage, pour fixer au moins une partie de prothèse dentaire (4a) au niveau de la section de gabarit (4).

12. Dispositif selon au moins l'une quelconque des revendications précédentes, où la section de référence (2), la section de gabarit (4) et les moyens de maintien (12) présentent un matériau sensiblement non représentable et les éléments de référence (10) et l'au moins une partie de prothèse dentaire (4a) présentent un matériau sensiblement représentable.

13. Dispositif selon au moins l'une quelconque des revendications précédentes, où la section de gabarit (4) présente sensiblement une mise en forme incurvée de manière à correspondre à la mâchoire (6).

14. Dispositif selon au moins l'une quelconque des revendications précédentes, où la section de gabarit (4) est prévue pour pratiquer au moins un élément fonctionnel (42) réalisé en tant qu'alésage de passage pour le logement d'une fouille de guidage de foret (44).

15. Dispositif selon au moins l'une quelconque des revendications précédentes, **caractérisé en ce que** la section de gabarit (4) présente des moyens de fixation, en particulier des broches d'ancrage, destinés à être fixés de manière amovible selon un agencement défini au niveau de la mâchoire du patient.

16. Procédé pour fabriquer une structure d'implant dentaire en utilisant un dispositif selon au moins l'une quelconque des revendications précédentes, avec les étapes :
A) de fourniture à la section de gabarit (4) d'au moins une partie de prothèse dentaire,
B) de production avec le système de balayage (20) de données d'image de la mâchoire d'un patient avec la section de gabarit (4) fixée au niveau de celle-ci,
C) de définition, sur la base des données obtenues sur les données d'image pour les éléments de référence (10), d'un système de coordonnées de référence (R),
D) de production, au moyen du premier système de traitement (22) sur les données d'image pour arrêter la position, l'orientation et l'espace (50) pour le logement d'un implant, de données d'implant, qui indiquent des paramètres pour l'agencement de l'implant tels qu'en particulier les dimensions de l'espace (50) arrêté à cet effet,
E) de comparaison des données d'implant au moyen du deuxième système de traitement (26) à des données sauvegardées d'une pluralité de différentes réalisations d'implant et d'indication en tant que résultat de ladite comparaison d'au moins une réalisation d'implant appropriée issue de la pluralité des différentes réalisations d'implant,
F) de transformation des données d'implant au moyen du système de transformation (34) sur la base d'un système de coordonnées de référence (R) en des données de machine pour une machine d'usinage (38) à utiliser en lien avec l'insertion de l'implant dans la mâchoire,
G) de serrage dans une machine d'usinage (38) de l'ensemble composé de la section de gabarit (2) et la section de référence (4) avec les moyens de maintien (12), et
H) de pratique, à l'aide de la machine d'usinage (38), en utilisant les données de machine, de l'au moins un élément fonctionnel (42), de manière préférée un alésage, au moins dans la section de gabarit (4).

17. Procédé selon la revendication 16, où, dans l'étape D, les données d'implant sont produites en tenant compte d'une distance minimale prédéfinie par rapport à un obstacle adjacent tel qu'en particulier une dent, un implant et/ou un nerf adjacent(e) et/ou par rapport aux côtés extérieurs de la mâchoire de sorte qu'elles définissent pour l'agencement d'un implant seulement un espace (50) tel, qu'il se situe à une distance par rapport à un obstacle adjacent ou aux côtés extérieurs de la mâchoire, laquelle correspond au moins à la distance minimale prédéfinie.

18. Procédé selon la revendication 16 ou 17, où, dans l'étape E, un groupe de réalisations d'implant appropriées est indiqué en tant que résultat de la comparaison parmi la pluralité des différentes réalisations d'implant aux fins de la sélection par l'utilisateur.

19. Procédé selon au moins l'une quelconque des revendications 16 à 18, où les données d'implant sont adaptées de manière correspondante en tenant compte des données de la réalisation d'implant appropriée entre l'étape E et l'étape F.
